# EUROPEAN PATENT APPLICATION

(11) **EP 0 532 328 A2**
(43) Date of publication of application: **17.03.1993**
(21) Application number: 92308250.7
(22) Date of filing: 10.09.1992
(51) Int. Cl.: C07C 69/013, C07C 67/14, A61K 31/225, C07D 317/70, A61K 31/335

(54) **Novel lignans, intermediates thereof and a process for preparing the intermediates**

(30) Priority: 13.09.1991 JP 262823/91
(71) Applicant: TSUMURA & CO., Chuo-ku, Tokyo 103 (JP)
(72) Inventor: Kimura, Masayuki c/o Tsumura & Co, Inashiki-gun Ibaraki-ken (JP); Hosaka, Kunio c/o Tsumura & Co, Inashiki-gun Ibaraki-ken (JP); Mitsuhashi, Hiroshi c/o Tsumura & Co, Inashiki-gun Ibaraki-ken (JP)
(74) Representative: Holmes, Michael John

(57) **Abstract**

The present invention aims to provide derivatives of compound, said compound being represented by the following constitutional formula :

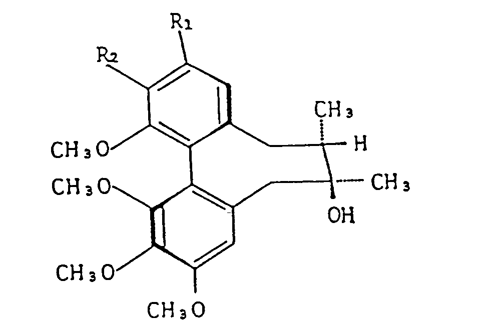

wherein R₁ and R₂ are OCH₃ or are joined to form -OCH₂0-. Said derivatives are lignan derivatives represented by the constitutional formula : 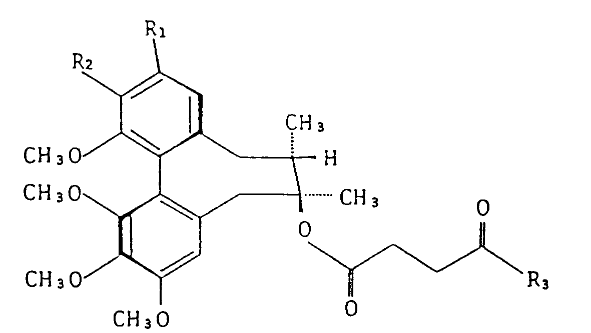 wherein R₁ and R₂ are as defined above, R₃ is a hydroxyl group, a hydroxyl group of which the hydrogen atom is substituted by a pharmaceutically acceptable metal, or the residual group of an amino acid or a pharmaceutically acceptable salt thereof.

## Description

### Technical Field of Invention

This invention relates to novel lignans, intermediates thereof, and a process for preparing the intermediates.

### Background Art

Compounds represented by the constitutional formula: 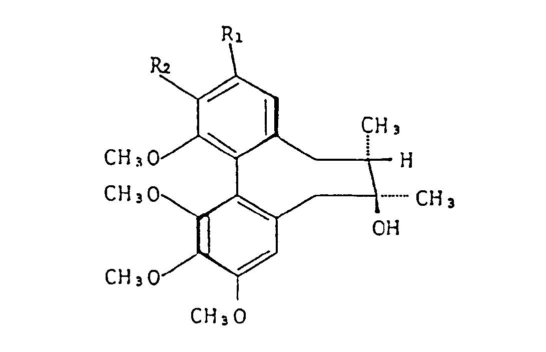
wherein R₁ and R₂ are OCH₃ or are joined to form -OCH₂0- are known. These compounds are extracted and isolated from Kita-Gomisi, which is obtained by drying the fruit of Korean Gomisi (Schizandra chinensis Baill) belonging to the Schisandraceae family.

Among the compounds represented by the formula above the compound in which R₁ and R₂ are OCH₃ is called shizandrin. The process for preparing it is described by N.K. Kochetkov et al., Zh. Obshch. Khim., 31, page 3454 (1961).

Among the compounds represented by the above formula, the compound in which R₁ and R₂ are joined to form -OCH₂0- is called Gomism A. A process for preparing it is described by Taguchi et al., Chem. Pharm. Bull. (Tokyo), 23, page 3296 (1975), and Taguchi et al., Chem. Pharm. Bull. (Tokyo), 25, page 364 (1977).

An improved process for preparing both of these compounds is described in Japanese Patent Early-publication No. 2-48592.

It is known that the compounds that are represented by said formulae show an antipyretic action and a hepatic lesion healing and that they are useful for antipyretics and therapeutic agents for hepatic failure (Japanese Patent Publication No. 60-33086 and Japanese Patent Early-publication No. 61-282315). It is known that said compounds are used as drugs for oral administration in the form of granules, tablets, capsules, etc., and for intravenous drip infusion (Japanese Patent Publication No. 60-33086 and Japanese Early-publication No. 61-282315). Japanese Patent Publication No. 3-27530 discloses that shizandrin is useful for brain function- improving medicines. Example 7 of said publication describes a process for preparing water-insoluble parenteral injections for which schizandrin is used for an injection other than an intravenous injection.

However, intravenous administrations are necessary to promptly produce drug effects in acute phase symptoms in hepatic lesion, etc., or to lower effective doses. An administration by intravenous drop infusion is not preferable as it causes patients physical and psychological pain. However, due to the low solubility in water (0.15 mg/ml of water), an aqueous injection faces a difficulty in maintaining an effective amount of the compound dissolved in an adequate amount of liquid for a one-time administration, because the amount of liquid per dose of the injection is limited to 10 to 20 ml. To make the amount of liquid adequate for the administration of an amount of the effective compound, a large amount of a solubilizing agent such as polyethylene glycol must be used. However, since this causes adverse effects, such as a hemolysis reaction, the amount used should be limited to a certain extent. Thus the development of a material having medicinal effects equivalent to said compounds and a proper solublity in water, as a substance for intravenous injection, has been sought.

The present inventors have conducted research over an extensive range regarding the derivatives of shizandrin and gomisin A. As a result they found that among novel lignans represented by the constitutional formula: 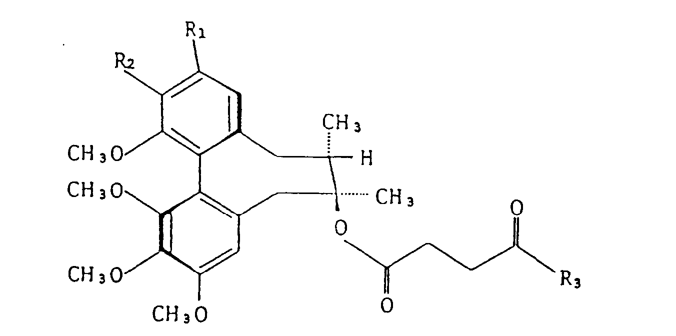
wherein R₁ and R₂ are as defined above and R₃ is a hydroxyl group, a hydroxyl group obtained by substituting a pharmacologically acceptable metal for a hydrogen atom, or an amino acid. residual group or a pharmacologically acceptable salt thereof, the salts of the lignans have a sufficient solubility in water and a similar medicinal effect to that of schizandrin and gomisin A.

### Disclosure of the Invention

The lignans of the present invention may be prepared as described in the following:
A metal hydride such as potassium hydride is saponified in a solvent to form a suspension. A compound (said compound is hereafter referred to as compound I) represented by the constitutional formula: 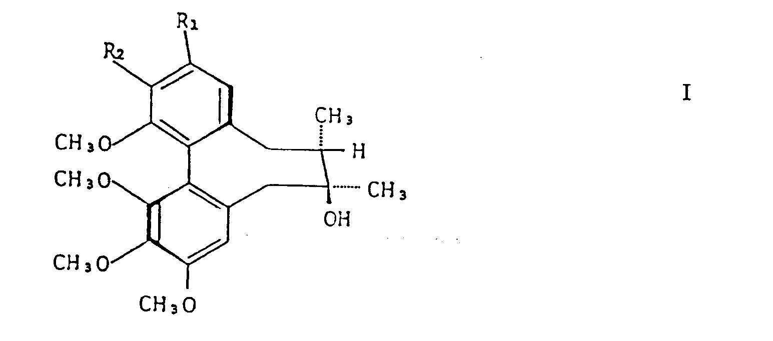
   wherein R₁ and R₂ are as defined above, is added to the thus-obtained suspension, and a metal alcoholate was formed. A base and 3-carbomethoxypropionyl chloride are successively added to the thus-formed alcoholate, the reaction mixture being heated to 75 to 85°C, and reacted. After cooling the reaction mixture to room temperature, it is extracted with an organic solvent and then sodium bicarbonate-saturated water is added to the thus-obtained organic solvent layer, or sodium bicarbonate-saturated water is added to the reaction mixture and then the mixture is extracted with an organic solvent. The thus-obtained extract is washed with water and a saturated salt solution, and dried over anhydrous magnesium sulfate, and the solvent is distilled off from the extract, thereby to obtain a novel lignan represented by the constitutional formula: 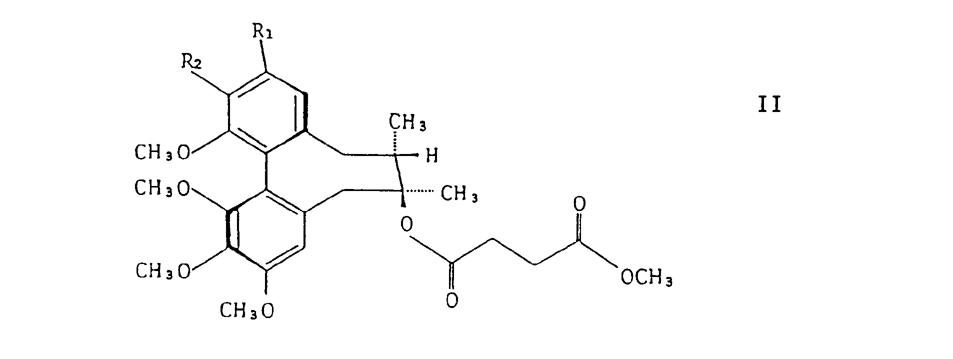
   wherein R₁ and R₂ are as defined above (said lignan hereafter is referred to as compound II). For the solvent to suspend the hydrogenated metal, hexamethylphosphoric triamide (HMPA), 2-methoxyethyl ether, or a combination of an ether-type solvent other than 2-methoxyethyl ether and 1,4,7,10,13,16-hexaoxacyclooctadecane, which is commercially avavailable as 18-Crown-6 (prepared by Aldrich), is used. For the ether-type solvent, tetrahydrofuran, ethylether, and methylether, may be exemplified. Although 1,4,7,10,13,16,-hexaoxacyclooctadecane is preferable because of its reactivity, it poses a problem because it is expensive. 2-methoxyethyl ether is preferable since the use thereof can remarkably increase the yield without using 1,4,7,10,13,16-hexaoxacyclooctadecane. 2-methoxyethyl ether is commercially available as diglyme, which is prepared by Aldrich. For the base to be added, pyridine, dimethylaminopyridine, 2,6-lutidine, or an amine-type base such as dimethyl aniline, may be used. 2,6-lutidine and dimethylaniline are preferable since they inhibit the production of the mixture of the three olefin compounds represented by these formulas:

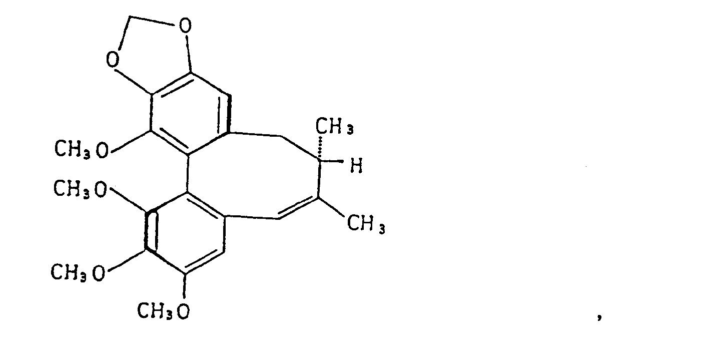

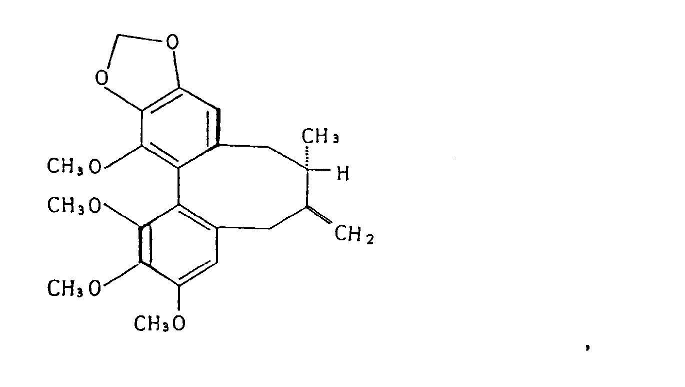

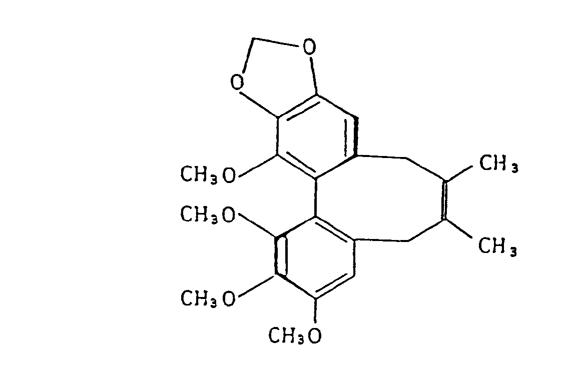

Dimethylaniline is especially preferable since it is cheaper and treatment upon its synthesis can be readily made.

If the amount of the metal hydride is high, danger of ignition exists on post-treatment of the reaction. Thus, it is necessary to decrease the number of the equivalent amount of the metal hydride as much as possible. Although the ratio of potassium hydride, dimethylaniline, and 3-carbomethoxypropyonyl chloride to compound I may be in a wide range, if the above point and yields of the objective products are taken into consideration, 1.1:5:5 is preferable.

For the extracting organic solvent, ethyl acetate, benzene, chloroform, or methylene chloride, is used.

The obtained compound II is dissolved in a solvent such as acetone and methanol. A dilute solution such as a 10 % or 5 % solution of sodium hydroxide or potassium hydroxde is added to the solution of the compound II to hydrolyze it. After washing the thus-obtained water layer with ethyl acetate etc. and adjusting it with a diluted hydrochloric acid to be acidic at about pH 2, the solution of the compound is extracted with ethyl acetate, dried over anhydrous magnesium sulfate, and the solvent is distilled off to obtain a novel compound represented by the constitutional formula: 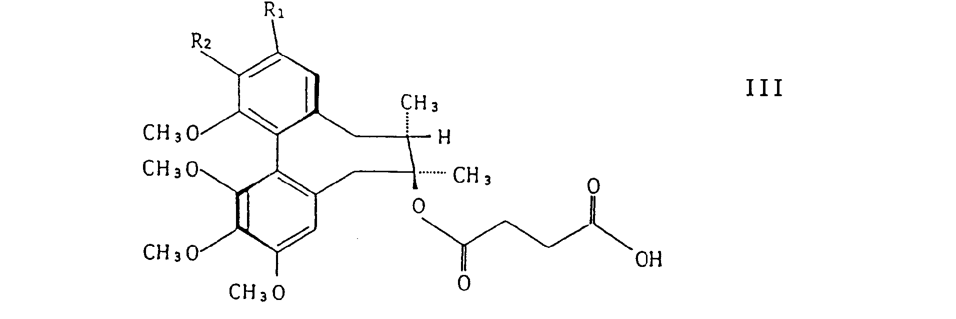
wherein R₁ and R₂ are as defined above, said compound being hereafter referred to as compound III.

A novel water-soluble lignan compound of the present invention (compound IV), represented by the constitutional formula: 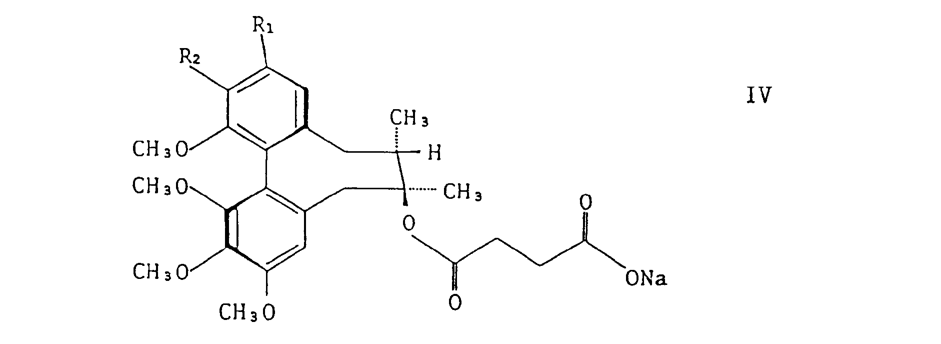
wherein R₁ and R₂ are as defined above, is obtained by dissolving compound III in methanol, reacting the solution with a methanol solution of sodium methoxide, or by dissolving compound III in a solvent such as methanol or acetone, adding a dilute solution of sodium hydroxide to that solution, and reacting them at room temperature, to obtain a precipitate that separates out. In this reaction, when instead of sodium methoxide a different pharmaceutically acceptable metal methoxide is used, and instead of the dilute solution of sodium hydroxide a dilute solution of a different pharmaceutically acceptable metal hydroxide is used, then instead of sodium half ester of succinic acid a pharmaceutically acceptable metal half ester of succinic acid is obtained. Said metals are, for example, potassium and calcium.

A compound represented by the constitutional formula: 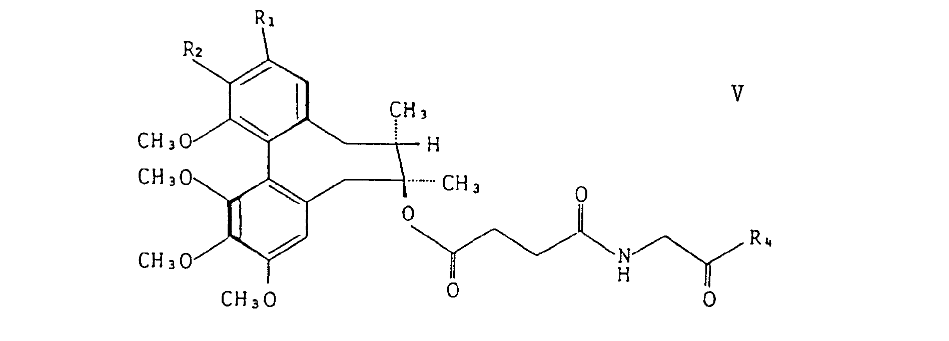
wherein R₁ and R₂ are as defined above and R₄ is a methoxyl group or a benzyloxy group, said compound being referred to as compound V, is obtained by dissolving compound III in a solvent such as methylene chloride, adding dicyclohexylcarbodiimide to the solution under ice-cooling, dehydrating and condensing the thus-obtained solution, adding a methyl ester or a benzyl ester of a free amino acid, subsequently adding ethyl acetate, removing the thus-resulting precipitate, and distilling off the ethyl acetate solvent. For the amino acid to be added, any amino acid, such as glycine, cystein, aspartic acid, or glutamic acid, may be used. For the solvent that dissolves compound III, methylene chloride (1.1 equivalent amount) is suitable, and compound V is obtained in a high yield by the above-reactions in methylene chloride at 0°C.

The thus-obtained compound V is dissolved in methanol, and a) when R₄ is a methoxyl group, a dilute water solution of sodium hydroxide is added to the solution to hydrolyze it, the thus-reacted solution is washed with ethyl acetate, and the water phase is concentrated, followed by acidifying with 10 % hydrochloric acid or b) when R₄ is a benzyloxyl group, hydrogen gas is added to compound V in the presence of palladium on carbon, the used palladium on carbon is removed, and the methanol is distilled off, to obtain a compound represented by the constitutional formula: 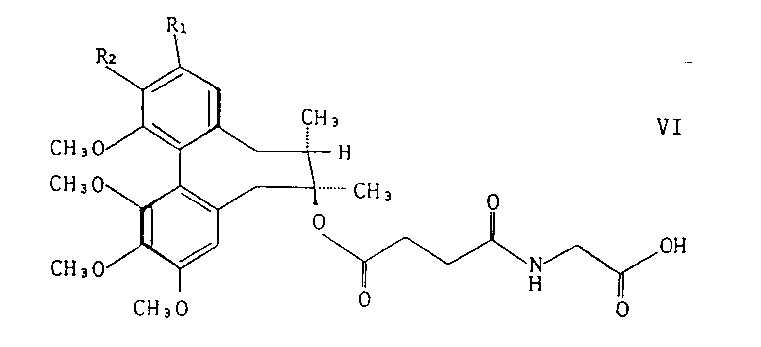
wherein R₁ and R₂ are as defined above, said compound being shown as compound VI. The thus-obtained compound VI is dissolved in a solvent such as acetone, the thus-obtained solution is reacted with a dilute water solution of sodium hydroxide, the thus-reacted solution is filtered to obtain a deposit, the thus-obtained deposit is washed with said solvent, and the thus-washed deposit is dried to obtain a novel lignan compound that is one of the present inventions and that is represented by the constitutional formula: 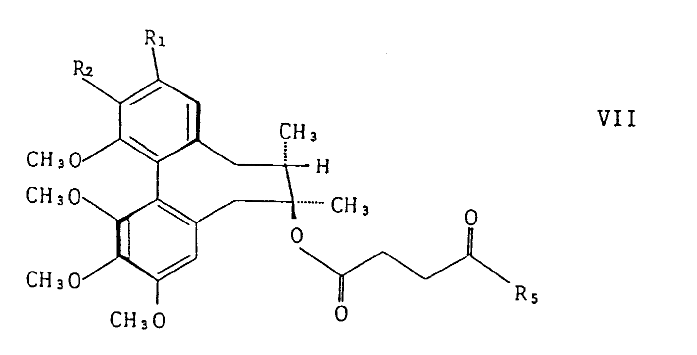
wherein R₁ and R₂ are as defined above and R₅ is a residual group of the sodium salt of an amino acid, said compound being referred to as compound VII. In this reaction, when instead of a dilute water solution of sodium hydroxide, a dilute water solution of another pharmaceutically acceptable metal hydroxide is used, then a compound is obtained wherein R₅ is, instead of the residual group of the sodium salt of amino acid, a pharmaceutically acceptable salt other than the residual group of the sodium salt of the amino acid. Said salts are, for example, a potassium salt and a calcium salt. It has been found that compounds IV and VII of the present inventions have an increased solubility in water. For example, the solubility of gomisin Asodium succinate represented by the constitutional formula: 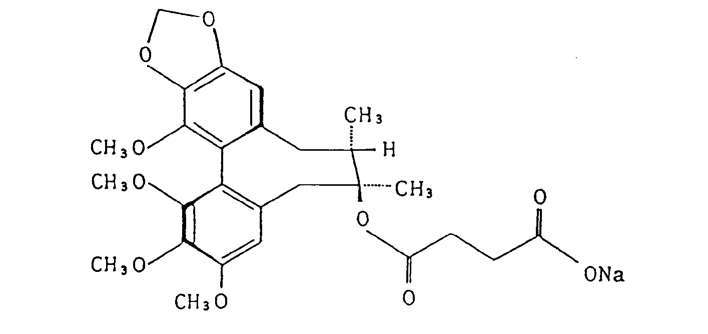
in purified water is 30 mg/2 ml, and was found to be 100 times the solubility of gomisin A. The solubility of gomisin A glycinosodium succinate represented by the constitutional formula: 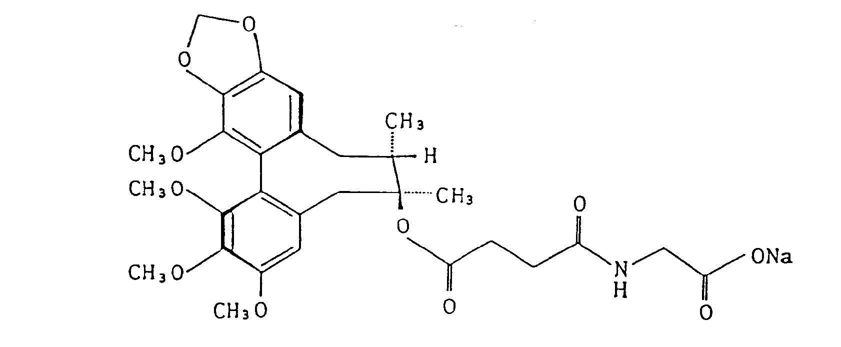
in a physiological salt solution was 30 mg/10 ml.

The solubility of schizandrin sodium succinate represented by the constitutional formula: 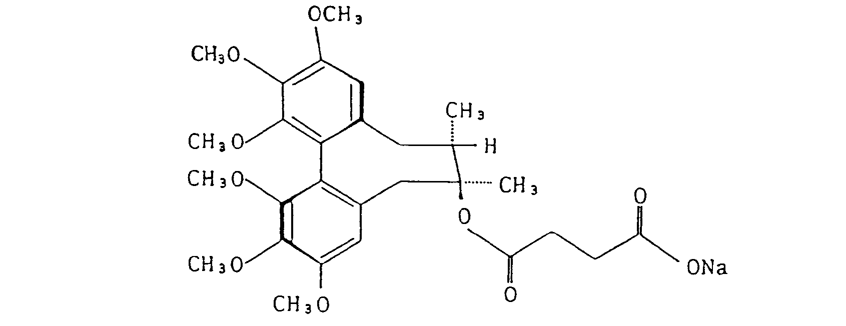
in purified water, the solubility of schizandrin glycinosodium succinate represented by the constitutional formula: 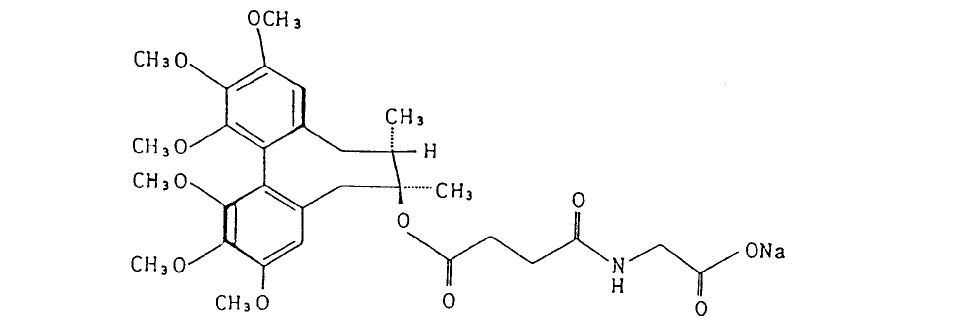
in purified water and in a physiological salt solution, were quite equivalent to the solubility of each corresponding derivative of gomisin A.

Both compounds IV and VII were found to have activities in tests of the improvement in hepatic lesion in the administration by intraperitoneal injection in SD strain male rats (7 weeks old).

### Example 1

1.76 g of hydrogenated potassium was suspended in 200 ml of diglyme in a 500 ml two-necked flask, then 17.28 g of schizandrin was added to the flask, and stirring was conducted at 50°C for one hour until the foaming stopped. Later 24.2 g of dimethylaniline was added to the flask, then 30.112 g of 3-carbomethoxypropionyl chloride was added, and the flask was warmed at 80°C for 20 hours. After leaving the flask to cool to room temperature, an extraction with ethyl acetate was conducted and then the extract was successively washed with water and a saturated salt solution. A saturated water solution of sodium bicarbonate was added to the ethyl acetate layer, stirring was conducted for one hour, the organic layer was separated, and then the thus-obtained layer was washed with a saturated salt solution and dried over anhydrous magnesium sulfate. After distilling off the solvent, schizandrin methyl succinate was obtained as a crude oily substance in the amount of 44.95 g.

The physical and chemical properties of the compound were as follows: Constitutional Formula: 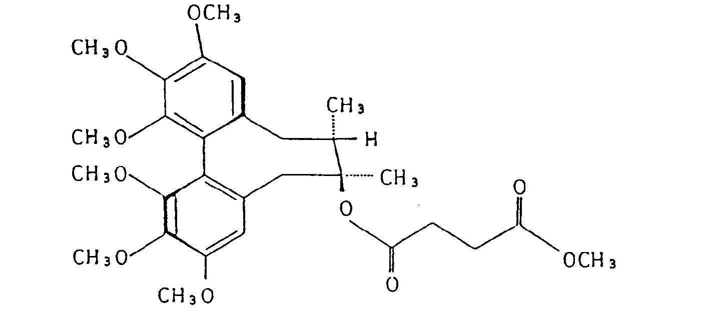
Molecular Formula: C₂₉H₃₈O₁₀
Molecular Weight: 546
Infrared Absorption Spectrum (KBr tablet method) νₘₐₓ cm-¹: 2940, 2836, 1722, 1592, 1574, 1492, 1462, 1452, 1430, 1402, 1360, 1338, 1320, 1268, 1240, 1198, 1154,1104,1070,1058,1034,1000,992
Nuclear Magnetic Resonance Spectrum(200 MHz) δ ppm (CDCI₃)₁ 0.84(3H, d, J=6.5), 1.58(3H, S), 1.70-1.85(1 H, m), 2.20-2.65(2H, m), 2.46(4H, S), 2.83(1 H, d, J=12), 2.95(1 H, d, J=12), 3.55(3H, s), 3.60(3H, s), 3.68(6H, s), 3.88(3H, s), 3.90(6H, s), 6.52(1 H, s), 6.68(1 H, s) Mass Spectrum m/z [El]:
546 (M⁺) 44.9 g of schizandrin methyl succinate was dissolved in 100 ml of acetone, 40 ml of 10 % sodium hydroxide was added to that solution, and then stirring at 55-60°C for two hours was conducted. After washing the solution with ethyl acetate, the pH of the solution was adjusted with 10 % hydrochloric acid to 2 and extraction with ethyl acetate (500 ml x 2) was conducted. After successively washing the extract with water and a saturated salt solution, the thus-obtained extract was dried over anhydrous magnesium sulfate. After the solvent was distilled off, schizandrin succinate was obtained as a pale yellow toffee-state substance in the amount of 28.5 g.

The physical and chemical properties of the compound were as follows:
Constitutional Formula: 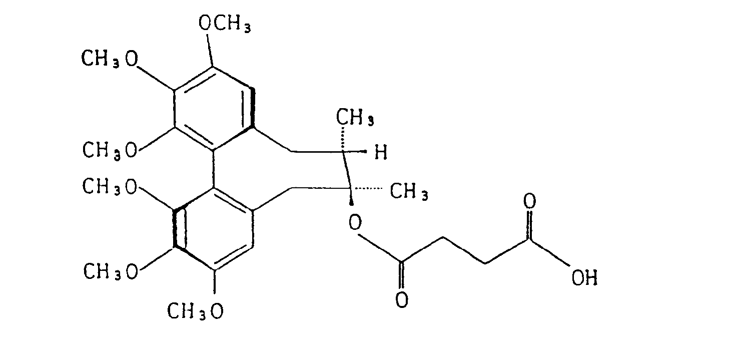
   Molecular Formula: C₂₈H₃₆O₁₀
   Molecular Weight: 532
   Infrared Absorption Spectrum (KBr tablet method) νₘₐₓ cm-¹: 3208, 2944, 2836, 1744, 1724, 1594, 1574, 1492, 1462, 1454, 1432, 1406, 1382, 1360, 1332, 1320, 1268, 1242, 1198, 1156, 1130, 1104, 1070, 1044, 1006, 992, 974, 926, 910, 832, 810
   Nuclear Magnetic Resonance Spectrum (200 MHz) δ ppm(CD₃0D): 0.845(3H, d, J=6.6), 1.601 (3H, s), 2.35-2.45(1 H, m), 2.423(4H, s), 2.558(1 H, d, J=7), 2.593(1 H, d, J=7), 2.834(1 H, d, J=12), 2.989(1 H, d, J=12), 3.464(3H, s), 3.497(3H, s), 3.846(3H, s), 3.859(3H, s), 3.886(3H, s), 4.853(1 H, s), 6.657(1 H, s), 6.749(1 H, s).
   Mass Spectrum m/z [HRP+FAB]: 532.2 (M⁺), 415.2
   Elemental Analysis Values: Calculated Values: C:63.14, H:6.81 Found Values: C:62.50, H:6.60

### Example 2

After dissolving 28 g of schizandrin succinate in 50 ml of methanol, a 28 % methanol solution of sodium methoxide (6.75 g) was added to the thus-obtained solution, and the thus-added solution was stirred at room temperature for five hours. After methanol was distilled off the obtained residue was washed with ether and dried under a reduced pressure to obtain schizandrin sodium succinate as pale brownish crystals in the amount of 18.75 g.

Total yield: 80 % (from schizandrin)

The physical and chemical properties of the compound were as follows:

Constitutional Formula: 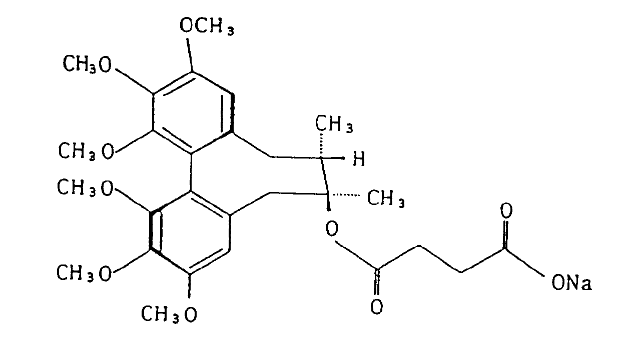
Molecular Formula: C₂₈H₃₅O₁₀Na
Molecular Weight: 554
Infrared Absorption Spectrum (KBr tablet method) νₘₐₓ cm-¹: 3440, 2940, 2836, 1718, 1594, 1574, 1492, 1462, 1430, 1402, 1360, 1340, 1322, 1268, 1240, 1198, 1156, 1104, 1072, 1034, 1008, 992, 954, 932, 840, 810, 732, 654
Nuclear Magnetic Resonance Spectrum (200 MHz) δ ppm(CD₃0D): 0.825(3H, d, J=6.5), 1.595(3H, s), 2.25.2.5(m, 6H), 2.551(1H, d, J=14), 2.848(1H, d, J=12), 3.002(1H, d, J=14), 3.442(3H, s), 3.458(3H, s), 3.830(3H, s), 3.836(3H, s), 3.876(6H, s), 6.688(1 H, s), 6.762(1 H, s). Mass Spectrum m/z [HRP+FAB]:
577.2 (M+Na)⁺, 437.2,163.0

### Example 3

After 342 mg of potassium hydride was suspended in 20 ml of diglyme in a 50 ml two-necked flask, 1.04 g of gomisin A was added to the flask and stirring was conducted at room temperature until the foaming stopped. Later 909 mg of dimethylaniline was added to the flask, then 1.13 g of 3-carbomethoxypropionyl chloride was added to it, and then the flask was warmed at 75-80°C for two days. After leaving the flask to cool to room temperature, 100 ml of a saturated water solution of sodium bicarbonate was added and stirring was conducted overnight. After extraction with 100 ml of ethyl acetate, the extract was successively washed with water and a saturated salt solution and dried over anhydrous magnesium sulfate. After the solvent was distilled off the thus-obtained crude oily substance was subjected to silica gel-flash column chromatography to obtain olefin material (4 %), gomisin A methyl succinate (73 %), and gomisin A (20 %).

The physical and chemical properties of the thus-obtained gomisin A methyl succinate are as follows: Constitutional Formula 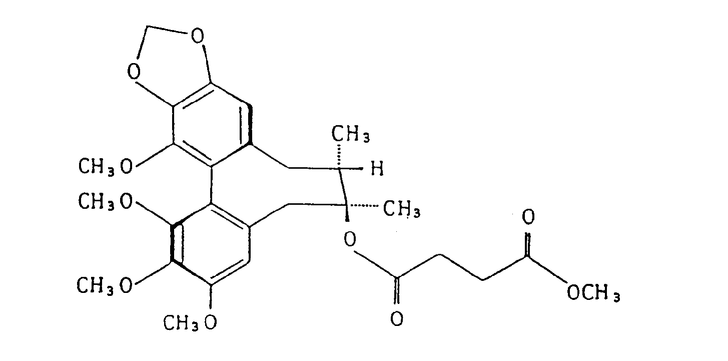
Molecular Formula: C₂₈H₃₀O₁₀
Molecular Weight: 530
Infrared Absorption Spectrum (KBr tablet method) νₘₐₓ cm-¹: 2936,1740,1728,1620,1598,1500,1462,1454,1420,1402,1368,1338,1270,1238,1198,1156,1106, 1048, 1020,998,954,934
Nuclear Magnetic Resonance Spectrum (200 MHz) δ ppm(CDCI₃): 0.814(3H, d, J=6.4), 1.587(3H, s), 2.438 (4H, s), 2.3-3.0(m, 5H), 3.513(3H, s), 3.645(3H, s), 3.804(3H, s), 3.868(3H, s), 3.890(3H, s), 5.945(1 H, d, J=1.5), 5.952(1 H, d, J=1.5), 6.454(1 H, s), 6.670(1 H, s).
Mass Spectrum m/z [EI] :
530 (M⁺, 14), 400(5), 399(28), 398(100), 383(12), 367(7), 352(5), 343(6), 337(6), 336(8), 181(16).

### Example 4

After 530 mg of gomisin A methyl succinate was dissolved in 1 ml of methanol and a 5 % methanol solution of 62 mg of potassium hydroxide was added to that solution, stirring was conducted at room temperature for one hour. After washing the thus-obtained solution with ethyl acetate, the pH of the solution was adjusted with 10 % hydrochloric acid to 2 and extraction with 50 ml of ethyl acetate was conducted. After succesively washing the thus-obtained extract with water and a saturated salt solution the thus-obtained extract was dried over anhydrous magnesium sulfate. After the solvent was distilled off, gomisin A succinate was obtained as pale yellow.low crystals in the amount of 507 mg. The crystals were purified by recrystallization, using ether, to obtain colorless crystals in the amount of 468 mg (yield: 91%).

### Example 5

After 3.43 g of potassium hydride was suspended in 80 ml of diglyme in a 500 ml two-necked flask, 4.16 g of gomisin A was added to the suspension and stirring was conducted at room temperature until foaming stopped. Later, after 3.635 g of aniline was added to the thus-obtained suspension, 4.5 g of 3-carbomethoxypropionyl chloride was added and heated at 75-80°C for two days. After leaving the flask to cool at room temperature, a saturated water solution of sodium bicarbonate was added to the reaction mixture and stirred for five hours. After extraction with ethyl acetate the extract was successively washed with water and a saturated salt solution and dried over anhydrous magnesium sulfate. After the solvent was distilled off, the obtained crude oily substance (6.9 g) was dissolved in 50 ml of methanol, 20 ml of 10 % sodium hydroxide was added, and the reaction mixture was stirred at room temperature for 30 minutes. After washing the water layer with ethyl acetate (200 ml), the pH of the water layer was adjusted with 10 % hydrochloric acid to 2, and then the water layer was extracted with 100 ml of ethyl acetate. After drying the extract over anhydrous magnesium sulfate, the solvent was distilled off to obtain a brownish oily substance in the amount of 4.9 g. The substance was separated and purified by silica gel-flash column chromatography to obtain gomisin A succinate in the amount of 3.62 g (yield: 70.2 %).

The physical and chemical properties of the compound were as follows:
Constitutional Formula: 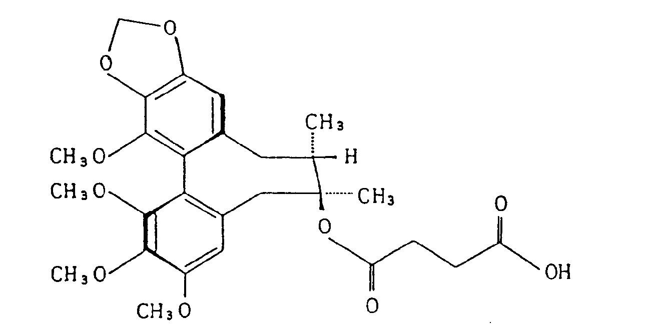
Molecular Formula: C₂₇H₃₂O₁₀
Molecular Weight: 516
Infrared Absorption Spectrum (KBr tablet method) νₘₐₓ cm-¹: 3208, 2936, 1740, 1714, 1622, 1598, 1500, 1480, 1462, 1422, 1402, 1372, 1338, 1296, 1270, 1238, 1196, 1153, 1150, 1110, 1082, 1048, 1036, 1018, 994, 938, 904, 846, 826, 766, 732, 716, 636.
Nuclear Magnetic Resonance Spectrum (200 MHz) δ ppm(CDCI₃):
0.814(3H, d, J=6.4), 1.586(3H, s), 2.25-2.95(m, 6H), 2.596(1H, d, J=14), 2.743(1 H, d, J=12), 2.921 (1 H, d, J=14), 3.503(3H, s), 3.812(3H, s), 3.857(3H, s), 3.891(3H, s), 5.951(1H, d, J=1.5), 5.965(1 H, d, J=1.5), 6.459(1 H, s), 6.660(1 H, s).
Mass Spectrum m/z [FD]: 517 (M+1), 399
Elemental Analysis Values: Calculated Values: C:62.78, H:6.24 Found-Values: C:62.63, H:6.19

### Example 6

After dissolving 516 mg of gomisin A succinate in 10 ml of acetone, a 10 % water solution of 33 mg sodium hydroxide was added to the thus-obtained solution and stirred at room temperature. Although at the beginning- of the reaction the solution was heterogeneous, after a while it became homogeneous. After the stirring was further continued, white turbidity began to occur, and the precipitate was separated out. The precipitate was filtered off, washed with acetone, and then dried under a reduced pressure to obtain gomisin A sodium succinate as colorless crystals in the amount of 510 mg (yield: 95 %).

The physical and chemical properties of the compound were as follows:
Constitutional Formula: 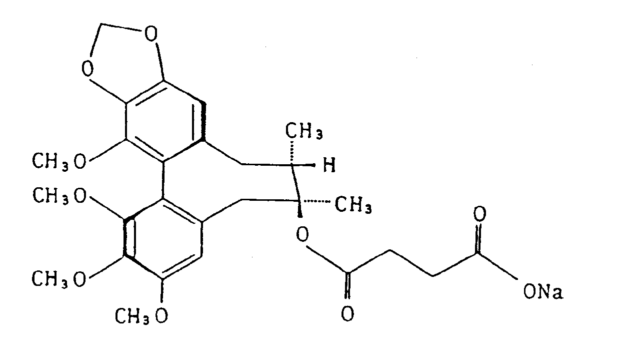
Molecular Formula: C₂₇H₃₁O₁₀Na
Molecular Weight: 538
Infrared Absorption Spectrum (KBr tablet method) νₘₐₓ cm-¹: 3432, 2976, 2940, 1712, 1620, 1600, 1576, 1196, 1156, 1108, 1036, 1048, 1018, 998, 952, 934, 864, 818, 732, 704, 650.
Nuclear Magnetic Resonance Spectrum (200 MHz) δ ppm(CD₃0D): 0.831(3H, d, J=6.4), 1.587(3H, s), 2.3-2.5(m, 6H), 2.595(1H, d, J=14), 2.763(1H, d, J=12), 2.920(1H, d, J=14), 3.432(3H, s), 3.746(3H, s), 3.863(3H, s), 3.883(3H, s), 5.950(1H, d, J=1.5), 5.964(1H, d, J=1.5), 6.510(1 H, s), 6,775(1 H, s).
Mass Spectrum m/z [FAB]:
539(M+H)⁺, 516(M+H-Na)

### Example 7

After ammonia was bubbled into 1.255 g of a commercially available hydrochloric acid salt of glycine methyl ester, 507 mg of free crude glycine methyl ester was obtained by ether extraction. After 2.064 g of gomisin A succinate was dissolved in 50 ml of methylene chloride and cooled to 0°C, 908 mg of diyclohexylcarbodiimide was added to the solution. After stirring at 0°C for 15 minutes, the earlier obtained free glycine methyl ester was added to the solution and further stirred at the same temperature for 30 minutes. 100 ml of ethyl acetate was added to the thus-obtained solution, the resulting precipitate was removed, and the solvent was distilled off. After 50 ml of additional ethyl acetate was added to the residue, the undissolved substance was removed, and the solvent was distilled off to obtain a toffee-state substance. The thus-obtained crude substance was subjected to silica gel-column chromatography to obtain 4-methylglycino-4-oxobutyryl gomisin Aas a colorless toffee-state substance in the amount of 2.25 g (yield: 90 %).

The physical and chemical properties of the compound are as follows:
Constitutional Formula: 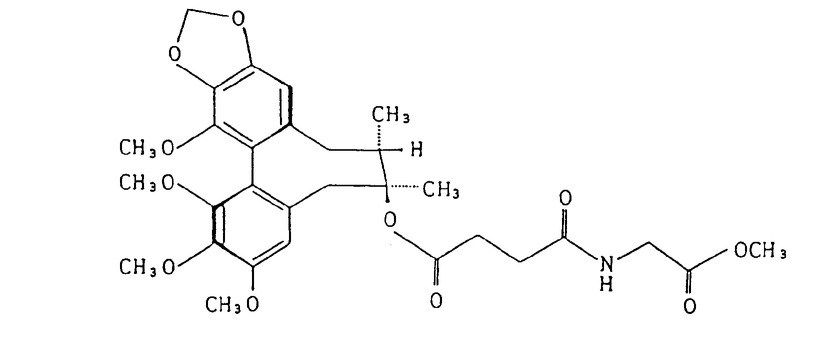
Molecular Formula: C₃₀H₃₇NO₁₁
Molecular Weight: 587
Infrared Absorption Spectrum (KBr tablet method) νₘₐₓ cm-¹: 3388, 2940, 1760, 1726, 1680, 1622, 1598, 1536, 1502, 1478, 1462, 1420, 1338, 1272, 1238, 1206, 1156, 1106, 1090, 1046, 1018, 998, 984, 952, 932, 904, 846, 818.
Nuclear Magnetic Resonance Spectrum (200 MHz) δ ppm(CDCI₃): 0.817(3H, d, J=6.8), 1.590(3H, s), 2.2-2.5(m, 5H), 2.60(1H, d, J=14), 2.74(1H, d, J=12), 2.92(1H, d, J=14), 3.514(3H, s), 3.752(3H, s), 3.814(3H, s), 3.876(3H, s), 3.897(3H, s), 4.057(2H, d, J=4.5), 5.951 (1 H, d, J=1.5), 5.966(1 H, d, J=1.5), 6.458(1 H, s), 6.685(1 H, s).
Mass Spectrum m/z [FD] :
587 (M⁺), 416

### Example 8

After 400 mg of a commercially available hydrochloric acid salt of glycine benzyl ester was alkalified with 10 % sodium hydroxide and extraction with ethyl acetate was made, 300 mg of free crude glycine benzyl ester was obtained. After 516 mg of gomisin A succinate was dissolved in 10 ml of methylene chloride and the solution was cooled to 0°C, 227 mg of diyclohexylcarbodiimide was added. After stirring at 0°C for 10 minutes the earlier obtained free glycine benzyl ester was added to the solution and stirring at the same temperature for an additional 10 hours was conducted. 5 ml of ethyl acetate was added to the thus-obtained solution, the resulting precipitate was removed, and the solvent was distilled off. After 5 ml of additional ethyl acetate was added to the residue and the unsoluble material was removed, the solvent was distilled off to obtain 715 mg of a toffee-state substance. The obtained toffee-state substance was subjected to silica gel-column chromatography to obtain 610 mg of 4-benzylglycino-4-oxiobutyryl gomisin A (yield: 92%).

The physical and chemical properties of the compound were as follows:
Constitutional Formula: 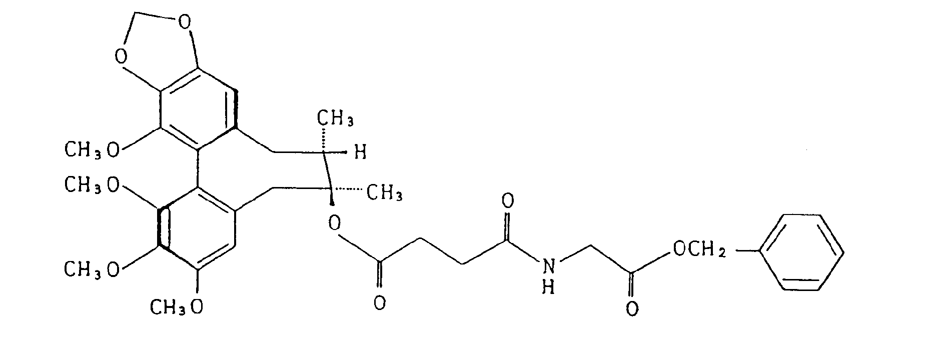
Infrared Absorption Spectrum (KBr tablet method) νₘₐₓ cm-¹: 3380, 2936, 1740, 1680, 1620, 1598, 1528, 1500, 1464, 1420, 1404, 1384, 1368, 1338, 1270, 1230, 1194, 1156, 1106, 1046, 1018, 998, 986, 952, 936, 906, 842, 818, 736.
Mass Spectrum m/z [FD]:
663 (M⁺)

### Example 9

450 mg of 4-benzylglycino-4-oxobutyryl gomisin A was dissolved in 10 ml of methanol and hydrogen gas was added to the thus-obtained solution in the presence of 50 mg of palladium on carbon. After the disappearance of the spot of the raw material was found by thin-layer chromatography, the used palladium on carbon was passed through celite to remove it and then the solvent was distilled off. Drying under a reduced pressure was conducted to obtain 4-glycine-4-oxobutyryl gomisin A as colorless crystals in the amount of 351 mg (yield: 90%).

The physical and chemical properties were as follows:
Constitutional Formula: 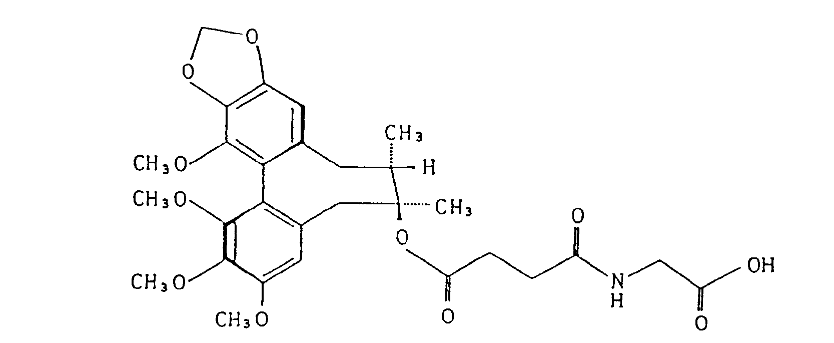
Molecular Formula: C₂₉H₃₅NO₁₁
Molecular Weight: 573
Infrared Absorption Spectrum (KBr tablet method) νₘₐₓ cm-¹: 3432, 2936, 1726, 1706, 1626, 1600, 1550, 1504, 1478, 1464, 1422, 1404, 1372, 1340, 1272, 1240, 1210, 1160, 1110, 1080, 1050, 1018, 998, 952, 932.
Nuclear Magnetic Resonance Spectrum (200 MHz) δ ppm(CDCI₃):
0.812(3H, d, J=6.9), 1.586(3H, s), 2.3-2.5(m, 6H), 2.598(1 H, d, J=14), 2.710(1 H, d, J=12), 2.878(1 H, d, J=14), 3.505(3H, s), 3.810(3H, s), 3.867(3H, s), 3.890(3H, s), 4.025(2H, ABq), 5.957(1 H, d, J=1.5), 5.974(1 H, d, J=1.5), 6.454(1 H, s), 6.670(1 H, s).
Mass Spectrum m/z [FD]: 573 (M⁺), 398

### Example 10

After 2 g of 4-methylglycino-4-oxobutyryl gomisin A was dissolved in 15 ml of methanol, and a 10 % water solution of 132 mg of sodium hydroxide (0.97 equivalent amount) was added to that solution, stirring was conducted at room temperature for 30 minutes. The solvent was distilled off and the residue was washed with ethyl acetate. The ethyl acetate-insoluble substance was dried under reduced pressure to obtain 4-glycine sodium-4-oxobutyryl gomisin A as colorless crystals in the amount of 1.854 g (yield: 92 %).

The physical and chemical properties were as follows:
Constitutional Formula: 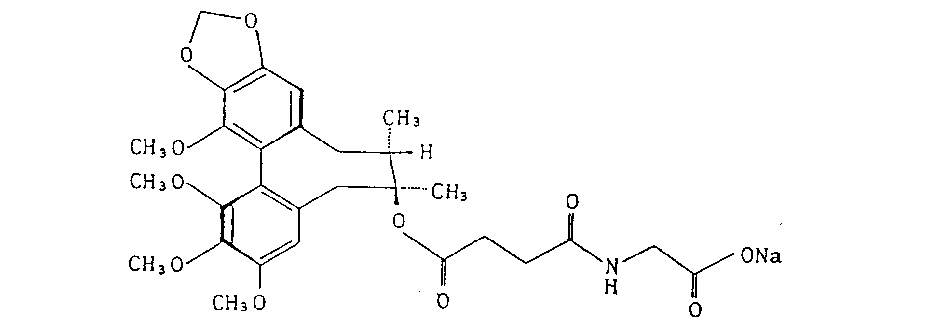
Molecular Formula: C₂₉H₃₄NO₁₁Na
Molecular Weight: 595
Infrared Absorption Spectrum (KBr tablet method) νₘₐₓ cm⁻¹: 3420, 2976, 2936, 1712, 1675-1600, 1502, 1480, 1462, 1454, 1420, 1402, 1372, 1340, 1238, 1272, 1238, 1196, 1158, 1110, 1084, 1060, 1048, 1018, 1000, 986, 954, 932, 820.
Nuclear Magnetic Resonance Spectrum (200 MHz) δ ppm(CD₃0D):
0.815(3H, d, J=6.6), 1.584(3H, s), 2.3-2.5(m, 3H), 2.418(4H, s), 2.562(1H, d, J=14), 2.767(1H, d, J=12), 2.929(1 H, d, J=14), 3.343(3H, s), 3.429(3H, s), 3.705(2H, s), 3.733(3H, s), 3.835(3H, s), 3.865(3H, s), 5.934(1 H, d, J=1.5), 5.947(1 H, d, J=1.5), 6.524(1 H, s), 6.756(1 H, s).
Mass Spectrum m/z [FAB] : 618 (M+Na)⁺, 596 (M+H)⁺

## Claims

1. Novel lignans represented by the constitutional formula: 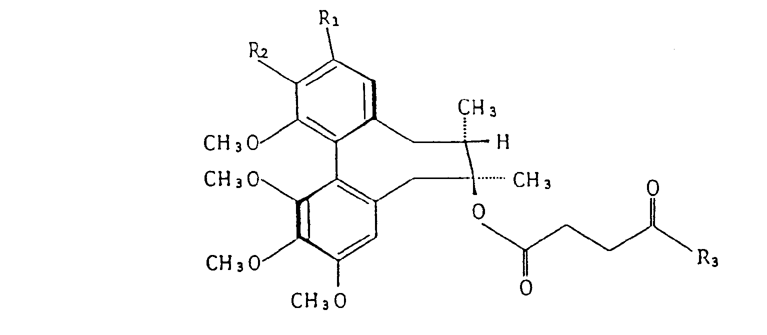
wherein R₁ and R₂ are OCH₃ or are joined to form-OCH₂O-, and R₃ is a hydroxyl group, or a hydroxyl group of which the hydrogen atom is substituted by a pharmaceutically acceptable metal, or the residual group of an amino acid or a pharmaceutically acceptable salt thereof.

2. Novel lignans represented by the constitutional formula: 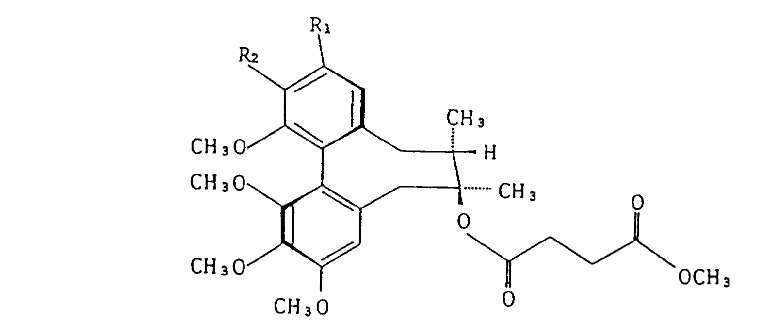
wherein R₁ and R₂ are OCH₃ or are joined to form -OCH₂0-3. A process for preparing novel lignans represented by the constitutional formula: 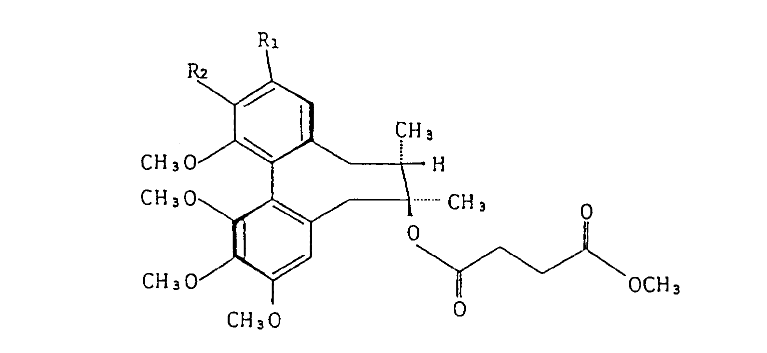
wherein R₁ and R₂ are OCH₃ or are joined to form -OCH₂0- by suspending a metal hydride in 2-methoxyethyl ether, adding to the thus-obtained suspension a compound represented by the constitutional formula: 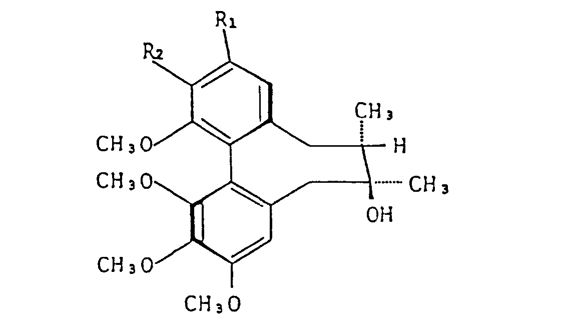
wherein R₁ and R₂ are as defined above, subjecting the mixture to a reaction, successively adding to the thus-obtained mixture an amine-type base and 3-carbomethoxypropionyl chloride, and subjecting the thus-obtained mixture to a reaction.

4. A pharmaceutical composition comprising a compound as defined in claim 1 together with at least one pharmaceutically acceptable carrier or excipient.

5. A lignan as defined in claim 1 for use as an antipyretic agent or an agent for the treatment of hepatic lesions.

6. Use of lignan as defined in claim 1 in the manufacture of an antipyretic agent or a medicament for treating hepatic lesions.
